# EUROPEAN PATENT SPECIFICATION

(11) **EP 2 863 834 B1**
(45) Date of publication and mention of the grant of the patent: **09.10.2019**
(21) Application number: 13756685.7
(22) Date of filing: 18.06.2013
(51) Int. Cl.: A61C 19/00, A61C 5/62, A61C 13/15, A61N 5/06, A61C 19/06

(54) **IRRADIATION DEVICE OF A PASTE-TYPE COMPOSITION**
BESTRAHLUNGSVORRICHTUNG EINER PASTENARTIGEN ZUSAMMENSETZUNG
DISPOSITIF D'IRRADIATION D'UNE COMPOSITION DE TYPE PÂTE

(30) Priority: 22.06.2012 IT MI20121098
(43) Date of publication of application: 29.04.2015
(73) Proprietor: Coswell S.p.A., 40050 Funo di Argelato (BO) (IT)
(72) Inventor: GUALANDI, Jacopo, I-40136 Bologna (IT); PASCUCCI, Paolo, I-06034 Foligno (PG) (IT)
(74) Representative: Porta & Consulenti Associati S.p.A.
(86) International application number: PCT/IB2013/054983
(87) International publication number: WO 2013/190457

(56) References cited:
- US-A1- 2005 026 103
- US-A1- 2008 118 887
- US-A1- 2010 003 636

## Description

The present invention relates to, in general, the technical field of devices for irradiating paste-type compositions. More specifically, the present invention relates to an irradiation device capable of irradiating a paste-type composition at the moment of application on a treatment site. The invention also relates to a cartridge for application on a treatment site of a paste-type composition to be irradiated, the cartridge comprising a tube of paste-type composition able to be removably associated with such an irradiation device.

Within the framework of the present description and in the subsequent claims, the term "paste-type composition" is used to indicate any composition in paste for use in oral hygiene, for example whitening and/or antibacterial toothpastes, and in the cosmetic or dermatological field, for example skin rejuvenating creams.

Within the framework of the present description and in the subsequent claims, the term "irradiation" is used to indicate light emission at a wavelength selected based on the specific effect to be obtained on the paste-type composition irradiated, for example photoactivation or sterilization of the paste-type composition or any alternative known treatment.

In recent years there has been increasingly common use of photodynamic therapy (PDT) in fields like dermatology, typically for treating skin pathologies, aesthetic medicine, for example for skin rejuvenation, up to dentistry, for example for teeth whitening.

The operating principle is always the same and consists of irradiating a photosensitive substance with a light source of specific wavelength. After the irradiation, the photosensitive substance photoactivates, i.e. becomes excited, modifying its properties, in such a way making them functional for the specific application.

Within the framework of the present description and in the subsequent claims, the term "photosensitive paste-type composition" is used to indicate a paste-type composition for use in oral hygiene and in the cosmetic or dermatological field that is capable of photoactivating when irradiated with a light source of suitable wavelength.

Within the framework of the present description we will also refer in particular to an irradiation device for the photoactivation of a photosensitive paste-type composition for use in oral hygiene and to a cartridge for application, on a treatment site, of a photosensitive paste-type composition to be photoactivated for teeth whitening. However, it should be understood that an irradiation device and an application cartridge of an entirely analogous type can also be used for the application of paste-type compositions for dermatological use for skin rejuvenation, as well as for the paste-type compositions application for sterilization.

Teeth whitening techniques through irradiation of a photosensitive substance currently used are of three types: professional whitening techniques, home whitening techniques and combined whitening techniques.

Professional whitening consists of applying onto the teeth of a subject suitable whitening substances, typically based on high-concentration hydrogen peroxide or carbamide peroxide, and photoactivating them by irradiating them with an external energy source, typically consisting of a lamp that emits ultraviolet light at a specific wavelength.

Home whitening foresees a preliminary step of making a transparent mask that reproduces the shape of the subject teeth and it is constructed so as to leave a gap between the surface of the teeth and the mask itself. The subject then applies a gel supplied by the dentist onto the teeth and then wears the mask for a certain number of hours during the day or all night.

Finally, combined whitening consists of a combination of professional whitening and home whitening briefly described above. In practice, it involves performing a professional whitening sitting in a dental surgery and continuing the treatment using the aforementioned mask in the ways indicated above with reference to home whitening.

The techniques described above, however, have some drawbacks. First of all they all require that the dentist performs a check on the subject. Indeed, specialised personnel are necessary to manipulate both the photoactivated substance and the photoactivating light sources used in the professional technique, as well as to make the mask of the home technique. Secondly, such techniques often require bulky and expensive devices.

Document US 2005/0026103 discloses a process of whitening a tooth including the steps of providing a mechanical dispensing device of a predetermined amount of a whitening composition to an applicator tip; dispensing the amount of whitening composition to the applicator tip; applying the amount of whitening composition disposed on the applicator tip to the tooth to be whitened; and allowing the whitening composition to remain in contact with the tooth for a period of time sufficient to whiten the tooth.

In one embodiment, a light source is disposed on the mechanical dispensing device such that light emitted from the light source impinges on the whitening composition. For example, a light source may be disposed on the mechanical dispensing device such that light emitted from the light source impinges on whitening composition in a reservoir of the device and/or which has been dispensed onto the applicator tip.

Document US 2008/0118887 discloses a dental handgrip for delivering filling compound into a tooth cavity, the handgrip being provided with a connection device to which a container for the filling compound can be connected.

In some embodiments, the handgrip comprises a light emission device via which radiation having a wavelength and radiant power for hardening of filling material can be provided.

The dental handgrip can comprise a safety device to prevent a premature or inadvertent light emission onto the filling compound.

Document US 2010/0003636 discloses medical handpieces for dispensing filling material some of which comprise a connection device for a filling material container, a vibration unit for the generation and transmission of vibrations to the filling material and a conveying device for conveying filling material from the container.

The problem forming the basis of the present invention is that of providing an irradiation device of a paste-type composition and a cartridge for application on a treatment site of a paste-type composition to be irradiated for personal use, i.e. such as to allow a subject, for example, to whiten his teeth easily at his own home and in total safety.

Another object of the present invention is to provide an irradiation device of a paste-type composition and a cartridge for application on a treatment site of a paste-type composition to be irradiated, which are compact and easy to use, as well as efficient in terms of irradiation of the paste-type composition.

Consequently, the invention relates to an irradiation device of a paste-type composition and a cartridge for application on a treatment site of a paste-type composition to be irradiated according to the independent claims 1 and 12. Preferred characteristics are then reported in the dependent claims.

In the irradiation device according to the invention, the problems outlined above are solved by the particular configuration of the irradiation device itself. In fact, the paste-type composition is irradiated by the irradiation elements during its transit through the longitudinal through opening of the container body of the device, i.e. when being used. As a result there is irradiation of a limited amount of paste-type composition, with consequent increase in efficiency of irradiation. Moreover, the maximum hygiene and practicality of use is ensured.

According to the invention, the irradiation device comprises means for switching the at least one irradiation element on/off.

According to the invention, furthermore, the means for switching the at least one irradiation element on/off comprise an end portion extending from the container body and suitable for cooperating with a cap able to be removably associated with the dispensing head to switch on the at least one irradiation element at the time of decoupling of the cap from the dispensing head of the irradiation device. This advantageously allows to activate the irradiation elements only in the application step of the paste-type composition on the treatment site, with consequent energy saving and the operating life lengthening of the energy source.

Preferably, the container body is made from semi-transparent plastic material.

According to a preferred embodiment, the container body comprises a first substantially cylindrical portion and a second portion extending from the first portion.

Preferably, the second portion comprises a substantially cylindrical central part coaxial to the first portion and in which the longitudinal through opening is made and a frusto-conical part extending from the substantially cylindrical part.

Preferably, the frusto-conical part has one or more grooves at an inner surface thereof. Such grooves advantageously have a refraction effect of the light irradiated by the irradiation means.

Preferably, the first portion is made from opaque plastic material and the second portion is made from transparent plastic material.

Preferably, the dispensing head is threaded. This advantageously allows easy removable coupling between the irradiation device and the cap or other similar closing means.

Preferably, the longitudinal through opening has an end, opposite the one where the dispensing head extends, that is threaded for the removable coupling of the irradiation device with a tube containing the paste-type composition to be activated.

Preferably, the at least one irradiation element is a UV light emitting diode. This advantageously allows to sterilize the paste-type composition coming out from the irradiation device.

According to an alternative embodiment, the at least one irradiation element is a RGB light emitting diode. This advantageously allows to regulate the wavelength of light emission as a function of the particular composition to be irradiated and of the particular application for use of the irradiation device.

Preferably, the energy source consists of one or more normal or rechargeable batteries.

According to another aspect of the invention a cartridge is provided for application on a treatment site of a paste-type composition to be irradiated, the cartridge comprising a tube containing a paste-type composition to be irradiated and the aforementioned irradiation device of the paste-type composition, the tube of paste-type composition to be irradiated being able to be removably associated with the irradiation device according to the invention.

Preferably, the paste-type composition contained in the application cartridge is a photosensitive paste-type composition.

Further characteristics and advantages of an irradiation device of a paste-type composition and of a cartridge for application on a treatment site of a paste-type composition to be irradiated according to the invention will become clearer from the following description of their preferred embodiments, made with reference to the attached drawings. In such drawings:
- Figure 1 is a front view of an irradiation device of a paste-type composition according to a first preferred embodiment of the invention;
- Figure 2 is a front view of an illumination group of the irradiation device of Figure 1;
- Figure 3 is a plan view of the illumination group of the irradiation device of Figure 1;
- Figure 4 is a perspective view of a cartridge for application of a paste-type composition to be irradiated according to a preferred embodiment of the invention, with a cap coupled with the irradiation device;
- Figure 5 is a perspective view of the application cartridge of Figure 4, with the cap decoupled from the irradiation device;
- Figure 6 is an exploded perspective view, which schematically shows the cartridge according to the invention in the step of application on a treatment site of a photosensitive paste-type composition to be photoactivated;
- Figure 7 is a front section view of an irradiation device of a paste-type composition according to an alternative embodiment of the invention;
- Figures 8 and 9 are, respectively, an exploded view and an assembled view of the irradiation device of Figure 7, which show a particular way of using the irradiation device.

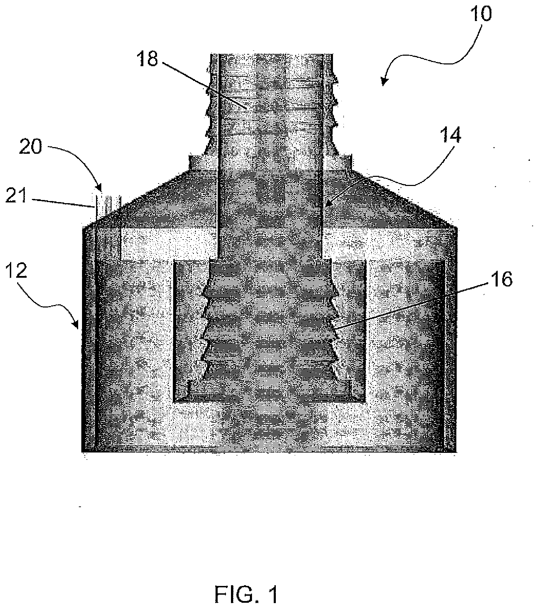

Figures 1 to 3 illustrate an irradiation device of a paste-type composition according to a first preferred embodiment of the present invention.

The irradiation device, indicated in general with reference numeral 10, comprises a container body 12, preferably substantially cylindrical, and an irradiation group 20 housed in the container body 12.

The container body 12 also has a longitudinal through opening 14 formed in it, coaxial to the container body 12, suitable for allowing the transit through the container body 12 of a paste-type composition to be irradiated with the irradiation device 10.

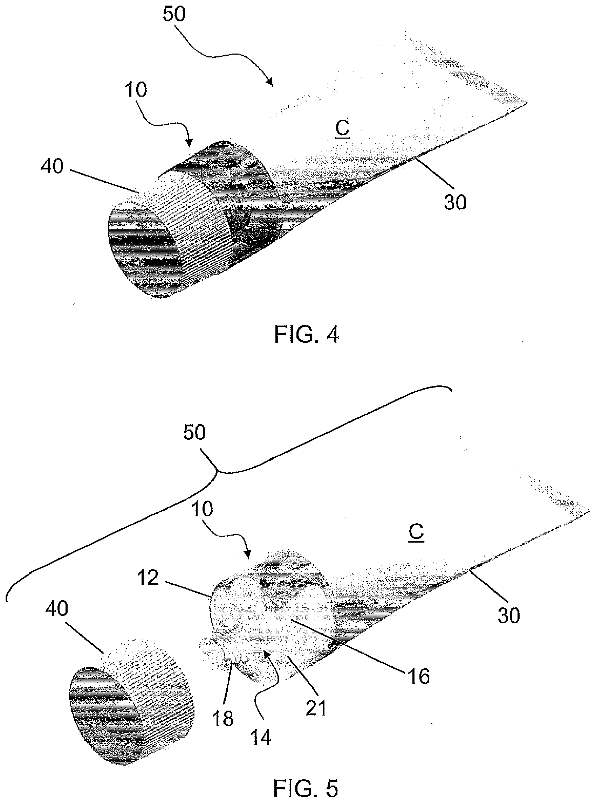

Preferably, the longitudinal through opening 14 has, at a lower end thereof 16, removable coupling means of the irradiation device 10 with a tube 30 (Figures 4-6) for containing the paste-type composition to be irradiated.

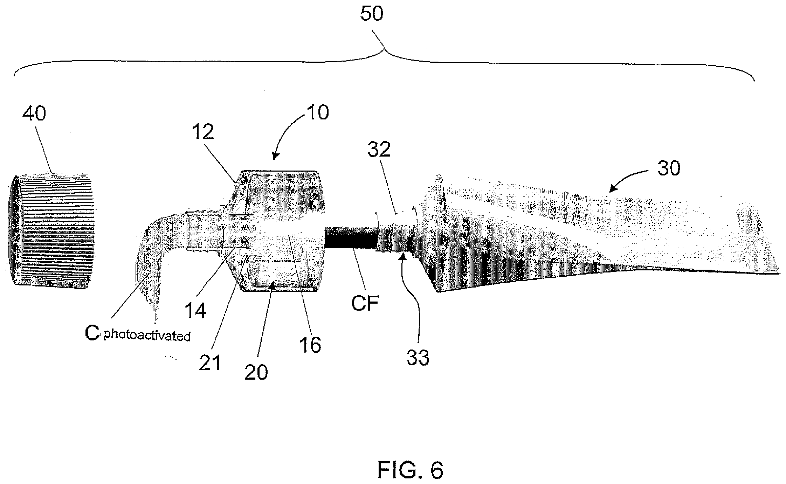

In the embodiment shown, the through opening 14 has a threaded lower end 16 on which it is possible to screw a corresponding threading 32 (Figure 6) formed at the application mouth 33 of the tube 30. Of course, between the irradiation device 10 and the tube 30 it is possible to foresee any other type of coupling suitable for the purpose, for example a snap coupling and the like.

Preferably, a dispensing head 18 extends from the longitudinal through opening 14 of the container body 12. Preferably, the dispensing head 18 is externally threaded so as to allow a cap 40 (Figures 4-6) or analogous closing means to be screwed in.

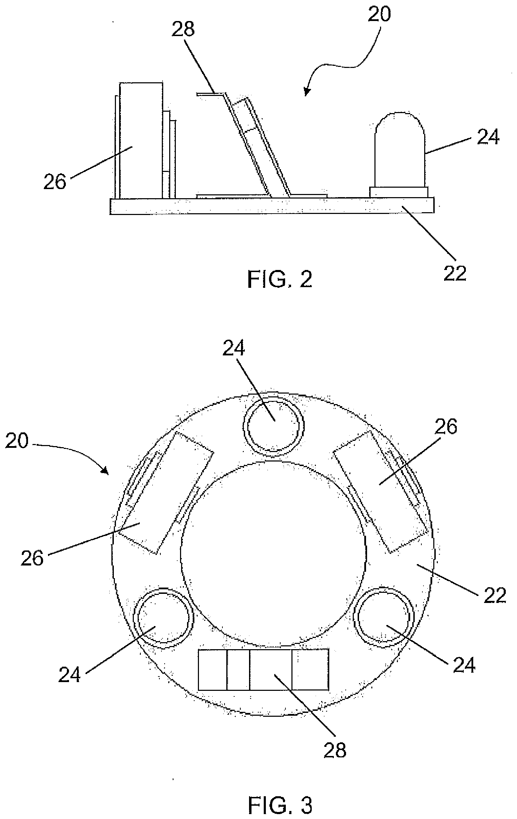

With reference to Figures 2 and 3, the irradiation group 20 comprises a preferably ring-shaped support base 22, on which one or more irradiation elements 24 are mounted, which are then positioned between the container body 12 and the longitudinal through opening 14. The irradiation group 20 further comprises an energy source 26 for the power supply of such irradiation elements 24.

Preferably, the irradiation elements consist of light emitting diodes (LEDs), for example LEDs that emit in the wavelengths of ultraviolet, RGB (Red Green Blue) LEDs and the like.

In particular, the use of UV LEDs advantageously allows to sterilize the paste-type composition C coming out from the photoactivator device 10, before being used, whereas the use of RGB LEDs advantageously allows to regulate the light emission over any wavelength necessary, for example blue light, depending on the different applications of the irradiation device.

The adjustment of the wavelength, and therefore of the colour emitted by the LEDs, takes place with the simple polarisation of the LEDs at the manufacturing stage of the irradiation device 10.

Preferably, the energy source comprises a pair of batteries 26, more preferably a pair of disposable batteries, in an absolutely preferred manner a pair of rechargeable batteries. Of course, the scope of protection of the invention covers all kinds of energy sources suitable for being mounted on the support plate 22 of the irradiation group 20.

According to the invention, the irradiation group 20 further comprises means 28 for switching the irradiation elements 24 on/off, for example a switch carried by the container body 12.

In the illustrated embodiment, and as can be seen in Figure 1, the means 28 for switching the irradiation elements 24 on/off comprise an end portion 21, the function of which will become clearer in the rest of the present description, which projects from the container body 12 of the irradiation device 10.

Figures 4 and 5 show a cartridge, indicated in general with reference numeral 50, for the application of a paste-type composition to be irradiated, on a treatment site, for example the teeth or the skin of a user.

In particular, the cartridge 50 comprises a tube 30 containing the paste-type composition C, for example a photosensitive composition to be photoactivated for teeth whitening or a paste-type composition to be sterilized, and the irradiation device 10, described above with reference to Figures 1 to 3, able to be removably associated with the tube 30. In particular, the irradiation device 10 is able to be removably associated with the tube 30 through screwing onto the application mouth 33 of the tube 30.

A cap 40 is able to be removably associated with the irradiation device 10 at its application mouth 18, preferably through screwing or another known removable coupling method.

With reference to Figures 4 to 6, the operation of the irradiation device 10 according to the present invention will now be described. In the described example the irradiation device 10 is used to photoactivate a photosensitive paste-type composition for teeth whitening. However, it should be understood that the same irradiation device 10 can be used in association with any other paste-type composition defined previously and for any kind of treatment that requires the irradiation of a paste-type composition, for example sterilization.

The irradiation device 10 is removably associated, for example screwed, at the lower end 16 of the longitudinal through opening 14 formed in the container body 12, with the application mouth 32 of a tube 30 containing a suitable photosensitive paste-type composition C to be photoactivated through irradiation. The irradiation device 10 is also removably associated, for example screwed, at its application mouth 18, with the cap 40.

A cartridge 50 is thus obtained for application of the photosensitive paste-type composition C on a treatment site, for example the teeth of a user in the case of teeth whitening.

In such an operating condition, the irradiation elements 24 of the irradiation group 20 are switched off, hence the irradiation device is deactivated. In particular, the cap 40 acts on the end portion 21 of the means 28 for switching the irradiation elements 24 on/off, interrupting the electrical connection circuit between the batteries 26 and the irradiation elements 24.

Let us now assume that a user wishes to apply an amount of photosensitive paste-type composition C on the treatment site. He will have to unscrew the cap 40 so as to decouple it from the irradiation device 10. After such an operation, the cap 40 no longer acts on the extreme end 21 of the means 28 for switching the irradiation elements 24 on/off. The electrical circuit between batteries and irradiation elements 24 thus closes, with consequent switching on of the latter.

By squeezing the tube 30, the user allows an amount of photosensitive paste-type composition C to pass through the longitudinal through opening 14 of the container body 12. During the transit through the opening 14, the photosensitive paste-type composition C is irradiated by the switched on irradiation elements 24, becoming photoactivated. The outlet mouth 18 of the irradiation device 10 thus lets out an amount of photoactivated paste-type composition C_{photoactivated}, which can easily be applied onto the teeth, for example with the help of a common toothbrush (not shown).

At the end of the operation, the user repositions the cap 40 on the irradiation device 10 taking it back into the rest condition, with the irradiation elements switched off.

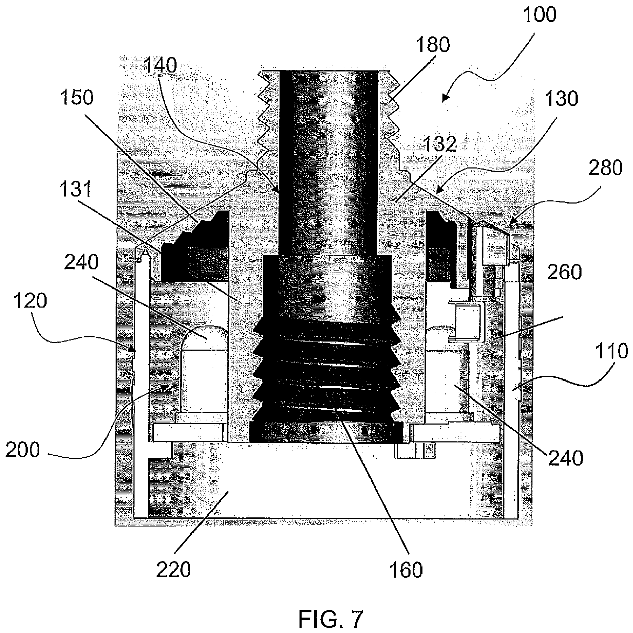

Figures 7 to 9 illustrate an irradiation device of a paste-type composition according to an alternative embodiment of the invention.

The irradiation device, indicated in general with reference numeral 100, comprises a container body 120 and an irradiation group 200 housed in the container body 120.

Preferably, the container body 120 comprises a first substantially cylindrical portion 110 and a second portion 130 extending from the first portion 110. In particular, the second portion 130 comprises a central part 131, also substantially cylindrical, coaxial to the first substantially cylindrical portion 110 and equipped with a longitudinal through opening 140, and a frusto-conical part 132 extending from the central part 131, for connecting to the first portion 110.

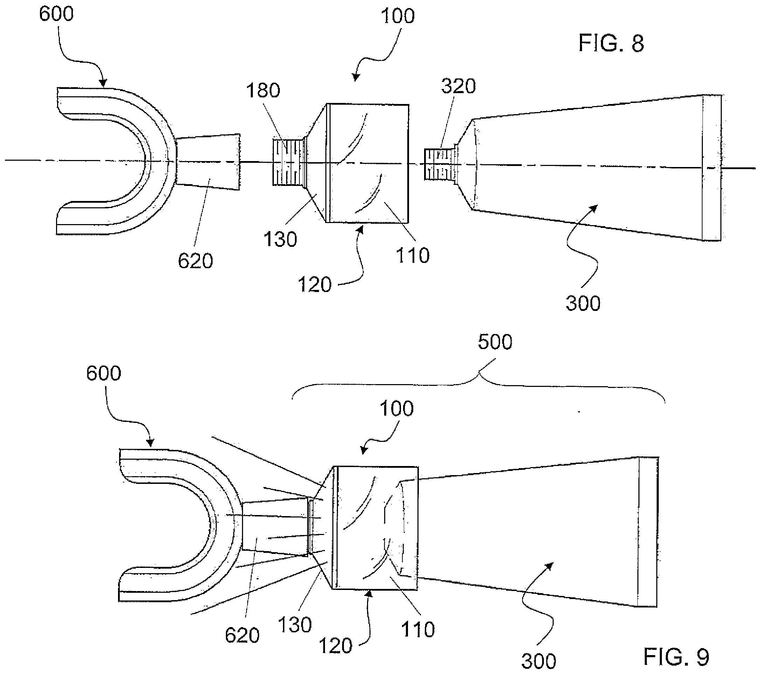

The longitudinal through opening 140 formed in the second portion 130 of the container body 120 is suitable for allowing the transit through the container body 120 of a paste-type composition to be irradiated with the irradiation device 100. The longitudinal through opening 140 also has a threaded lower end 160 on which it is possible to screw a corresponding threaded mouth 320 of a tube 300 containing the paste-type composition to be irradiated, as shown in Figures 8 and 9. The longitudinal through opening 140 has a dispensing head 180 extending from it, preferably externally threaded to allow a cap or similar closing means to be screwed onto it.

Preferably, the frusto-conical part 132 of the second portion 130 of the container body 120 has, at an inner surface thereof, one or more grooves 150, preferably circular, the function of which will become clearer in the rest of the present description.

Preferably, the first portion 110 of the container body 120 is made from opaque plastic material, whereas the second portion 130 of the container body 120 is made from transparent plastic material.

The irradiation group 200 comprises a preferably ring-shaped support base 220 on which one or more irradiation elements 240 are mounted, which are positioned between the first portion 110 of the container body 200 and the longitudinal through opening 140 formed in the second portion 130 of the container body 200. The support group 200 also comprises an energy source 260 for powering such irradiation elements 240.

The irradiation elements 240 and the energy source 260 are totally analogous to those described with reference to the first embodiment illustrated in Figures 1 to 6, for which reason they will not be detailed any further.

Preferably, the irradiation group 200 also comprises means 280 for switching the irradiation elements 240 on/off that are entirely similar to those of the embodiment illustrated in Figures 1 to 6, and that therefore will not be described any further.

As shown in Figures 8 and 9, the irradiation device 100 can be associated, for example through screwing, with a tube 300 containing a paste-type composition to be irradiated, so as to obtain a cartridge 500 for application on a treatment site of the paste-type composition.

Again with reference to Figures 8 and 9, they illustrate an alternative way of using the irradiation device 100 of Figure 7 in combination with a mask 600 for applying and laying a paste-type composition, for example with whitening effect, on the teeth of a user.

In particular, connection means between the mask 600 and the irradiation device 100 extend from the front of the mask 600, which in the embodiment is represented by cap 620 suitable for being screwed, in use, onto the threaded dispensing head 180 of the irradiation device 100.

The masks for applying and laying a whitening substance are known in the state of the art, for which reason they will not be described in detail in the present document.

Let us presume that a user wishes to whiten his teeth using the irradiation device 100 according to the invention in combination with a mask 600.

First of all he will have to apply a suitable amount of paste-type composition onto the mask 600, according to the methods described above with reference to Figures 4 to 6.

Thereafter, the user will have to connect the irradiation device 100 to the mask 600, for example by screwing the cap 620 onto the dispensing head 180 of the irradiation device 100, then fit the mask 600 onto the teeth.

The cap 620 is configured so as not to act on the means 280 for switching the irradiation elements 240 on/off, for which reason such irradiation elements are switched on and capable of emitting light at the wavelength necessary for the particular application.

The light irradiated by the irradiation elements 240 comes out from the frusto-conical part 132 of the transparent second portion 130 of the container body 120 and, through the effect of the presence of the circular grooves 150 formed in such a frusto-conical part 132, undergoes a refraction, so as to advantageously reach all of the points of the mask 600, and thus photoactivate all of the photosensitive paste-type composition applied onto the mask 600 itself.

Once the irradiation time necessary for whitening the teeth has passed, the user will extract the mask from his mouth, he will detach the irradiation device 100 from the mask 600 and he will put the irradiation device 100 back into the non-operative condition, with the irradiation elements 240 switched off.

Although the present invention has been described and illustrated with reference to specific preferred embodiments and to the specific application in the field of teeth whitening, it should be understood that the scope of protection defined by the following claims covers all of the possible variants within the capabilities of a man skilled in the art.

## Claims

1. Irradiation device (10; 100) of a paste-type composition (C) comprising:
- a container body (12; 120) equipped with a longitudinal through opening (14; 140) for the transit of a paste-type composition (C) to be irradiated and with a dispensing head (18; 180) extending from the longitudinal through opening (14; 140);
- at least one irradiation element (24; 240) arranged in the container body (12; 120) and suitable for irradiating the paste-type composition (C) in transit in the longitudinal through opening (14; 140); and
- an energy source (26; 260) also arranged in the container body (12; 120) and in electrical connection with the at least one irradiation element (24; 240); and
- means (28; 280) for switching said at least one irradiation element (24; 240) on/off;
**characterised in that** said means (28; 280) for switching said at least one irradiation element (24; 240) on/off comprises an end portion (21) extending from the container body (12; 120) and suitable for cooperating with a cap (40) able to be removably associated with the dispensing head (18; 180) of the irradiation device (10; 100) to switch on the at least one irradiation element (24; 240) at the time of decoupling of the cap (40) from the dispensing head (18; 180) of the irradiation device (10; 100)..

2. Device (10) according to claim 1, **characterised in that** said container body (12) is made from semi-transparent plastic material.

3. Device (100) according to claim 1 or 2, **characterised in that** said container body (120) comprises a first substantially cylindrical portion (110) and a second portion (130) extending from said first portion (110) .

4. Device (100) according to claim 3, **characterised in that** said second portion (130) comprises a substantially cylindrical central part (131) coaxial to the first portion (110) and in which said longitudinal through opening (140) is made and a frusto-conical part (132) extending from said substantially cylindrical part (131) .

5. Device (100) according to claim 4, **characterised in that** said frusto-conical part (132), at an inner surface thereof, has one or more grooves (150).

6. Device (100) according to any one of claims 3 to 5, **characterised in that** said first portion (110) is made from opaque plastic material and said second portion (130) is made from transparent plastic material.

7. Device (10; 100) according to any one of the previous claims, **characterised in that** said dispensing head (18; 180) is externally threaded.

8. Device (10; 100) according to any one of the previous claims, **characterised in that** said longitudinal through opening (14; 140) has an end (16; 160), opposite the one where the dispensing head (18; 180) extends, that is threaded for the removable coupling of a cartridge (30; 300) containing the paste-type composition (C) to be irradiated.

9. Device (10; 100) according to any one of the previous claims, **characterised in that** said at least one irradiation element (24; 240) is a light emitting diode.

10. Device (10; 100) according to claim 9, **characterised in that** said light emitting diode (24; 240) is a UV or RGB diode.

11. Device (10; 100) according to any one of the previous claims, **characterised in that** said energy source consists of one or more disposable or rechargeable batteries.

12. Cartridge (50) for application on a treatment site of a paste-type composition (C) to be irradiated, **characterised in that** it comprises a tube (30; 300) containing the paste-type composition (CF) to be irradiated and an irradiation device (10) according to any one of claims 1 to 11, said tube of paste-type composition being able to be removably associated with said irradiation device.

13. Cartridge (50) according to claim 12, **characterised in that** said paste-type composition (C) is a photosensitive paste-type composition.

## Patentansprüche

1. Bestrahlungsvorrichtung (10; 100) einer pastenähnlichen Zusammensetzung (C) umfassend:
- einen Behälterkörper (12; 120), der mit einer länglichen Durchtrittsöffnung (14; 140) für den Durchgang einer zu bestrahlenden pastenähnlichen Zusammensetzung (C) und mit einem sich von der länglichen Durchtrittsöffnung (14; 140) erstreckenden Ausgabekopf (18; 180) versehen ist;
- zumindest ein Bestrahlungselement (24; 240), das im Behälterkörper (12; 120) angeordnet ist und zur Bestrahlung der durch die längliche Durchtrittsöffnung (14; 140) gehenden pastenähnlichen Zusammensetzung (C) geeignet ist; und
- eine Energiequelle (26; 160), die ebenfalls im Behälterkörper (12; 120) angeordnet ist und mit dem zumindest einen Bestrahlungselement (24; 240) in elektrischer Verbindung steht; und
- Mittel (28; 280) zum Ein-/Ausschalten dieses zumindest einen Bestrahlungselements (24; 240);
**dadurch gekennzeichnet, dass** diese Mittel (28; 280) zum Ein-/Ausschalten dieses zumindest einen Bestrahlungselements (24; 240) einen sich vom Behälterkörper (12; 120) erstreckenden Endabschnitt (21) aufweisen, der zum Zusammenwirken mit einer Kappe (40) geeignet ist, die mit dem Ausgabekopf (18; 180) der Bestrahlungsvorrichtung (10; 100) abnehmbar verbunden werden kann, um das zumindest eine Bestrahlungselement (24; 240) zum Zeitpunkt des Entkoppelns der Kappe (40) vom Ausgabekopf (18; 180) der Bestrahlungsvorrichtung (10; 100) einzuschalten.

2. Vorrichtung (10) nach Anspruch 1, **dadurch gekennzeichnet, dass** dieser Behälterkörper (12) aus halbdurchsichtigem Kunststoff besteht.

3. Vorrichtung (100) nach Anspruch 1 oder 2, **dadurch gekennzeichnet, dass** dieser Behälterkörper (120) einen ersten im Wesentlichen zylindrischen Abschnitt (110) und einen sich von diesem ersten Abschnitt (110) erstreckenden zweiten Abschnitt (130) umfasst.

4. Vorrichtung (100) nach Anspruch 3, **dadurch gekennzeichnet, dass** dieser zweite Abschnitt (130) einen im Wesentlichen zylindrischen mittleren Teil (131), der koaxial zum ersten Abschnitt (110) ist, in welchem diese längliche Durchtrittsöffnung (140) ausgeführt ist, und einen sich von diesem im Wesentlichen zylindrischen Teil (131) erstreckenden kegelstumpfförmigen Teil (132) umfasst.

5. Vorrichtung (100) nach Anspruch 4, **dadurch gekennzeichnet, dass** dieser kegelstumpfförmige Teil (132) eine oder mehrere Nuten (150) auf eine seiner Innenoberflächen aufweist.

6. Vorrichtung (100) nach irgendeinem der Ansprüche von 3 bis 5, **dadurch gekennzeichnet, dass** dieser erste Abschnitt (110) aus undurchsichtigem Kunststoff besteht und, dass dieser zweite Abschnitt (130) aus durchsichtigem Kunststoff besteht.

7. Vorrichtung (10; 100) nach irgendeinem der vorgehenden Ansprüche, **dadurch gekennzeichnet, dass** dieser Ausgabekopf (18; 180) mit Außengewinde versehen ist.

8. Vorrichtung (10; 100) nach irgendeinem der vorgehenden Ansprüche, **dadurch gekennzeichnet, dass** diese längliche Durchtrittsöffnung (14; 140) ein Ende (16; 160) aufweist, das demjenigen gegenüberliegt, an welchem sich der Ausgabekopf (18; 180) erstreckt, der mit Außengewinde für die abnehmbare Verbindung einer Kartusche (30; 300) versehen ist, welche die zu bestrahlende pastenähnliche Zusammensetzung (C) enthält.

9. Vorrichtung (10; 100) nach irgendeinem der vorgehenden Ansprüche, **dadurch gekennzeichnet, dass** dieses zumindest eine Bestrahlungselement (24; 240) eine Leuchtdiode ist.

10. Vorrichtung (10; 100) nach Anspruch 9, **dadurch gekennzeichnet, dass** diese Leuchtdiode (24; 240) eine UV- oder RGB-Diode ist.

11. Vorrichtung (10; 100) nach irgendeinem der vorgehenden Ansprüche, **dadurch gekennzeichnet, dass** diese Energiequelle aus einer oder mehreren Einweg- oder wiederaufladbaren Batterien besteht.

12. Kartusche (50) zum Aufbringen an einen Behandlungsort einer zu bestrahlenden pastenähnlichen Zusammensetzung (C), **dadurch gekennzeichnet, dass** sie einen Schlauch (30; 300) umfasst, der die zu bestrahlende pastenähnlichen Zusammensetzung (CF) enthält und eine Bestrahlungsvorrichtung (10) nach irgendeinem der Ansprüche von 1 bis 11, wobei dieser Schlauch aus pastenähnlicher Zusammensetzung mit dieser Bestrahlungsvorrichtung lösbar verbunden werden kann.

13. Kartusche (50) nach Anspruch 12, **dadurch gekennzeichnet, dass** diese pastenähnlichen Zusammensetzung (C) eine photosensitive pastenähnliche Zusammensetzung ist.

## Revendications

1. Dispositif d'irradiation (10 ; 100) d'une composition de type pâte (C) comprenant :
- un corps de contenant (12 ; 120) équipé d'une ouverture traversante longitudinale (14 ; 140) pour le transit d'une composition de type pâte (C) à irradier et d'une tête de distribution (18 ; 180) s'étendant à partir de l'ouverture traversante longitudinale (14 ; 140) ;
- au moins un élément d'irradiation (24 ; 240) agencé dans le corps de contenant (12 ; 120) et conçu pour irradier la composition de type pâte (C) en transit dans l'ouverture traversante longitudinale (14 ; 140) ; et
- une source d'énergie (26 ; 160) également agencée dans le corps de contenant (12 ; 120) et en connexion électrique avec l'au moins un élément d'irradiation (24 ; 240) ; et
- des moyens (28 ; 280) pour activer/désactiver ledit au moins un élément d'irradiation (24 ; 240) ;
**caractérisé en ce que** lesdits moyens (28 ; 280) pour activer/désactiver ledit au moins un élément d'irradiation (24; 240) comprennent une partie d'extrémité (21) s'étendant à partir du corps de contenant (12 ; 120) et conçue pour coopérer avec un capuchon (40) apte à être associé de manière amovible à la tête de distribution (18 ; 180) du dispositif d'irradiation (10 ; 100) pour activer l'au moins un élément d'irradiation (24 ; 240) au moment du découplage du capuchon (40) de la tête de distribution (18 ; 180) du dispositif d'irradiation (10 ; 100).

2. Dispositif (10) selon la revendication 1, **caractérisé en ce que** ledit corps de contenant (12) est constitué de matière plastique semi-transparente.

3. Dispositif (100) selon la revendication 1 ou 2, **caractérisé en ce que** ledit corps de contenant (120) comprend une première partie sensiblement cylindrique (110) et une seconde partie (130) s'étendant à partir de ladite première partie (110).

4. Dispositif (100) selon la revendication 3, **caractérisé en ce que** ladite seconde partie (130) comprend une partie centrale sensiblement cylindrique (131) coaxiale à la première partie (110) et dans laquelle ladite ouverture traversante longitudinale (140) est ménagée et une partie tronconique (132) s'étendant à partir de ladite partie sensiblement cylindrique (131).

5. Dispositif (100) selon la revendication 4, **caractérisé en ce que** ladite partie tronconique (132), au niveau d'une surface interne de celle-ci, possède une ou plusieurs gorges (150).

6. Dispositif (100) selon l'une quelconque des revendications 3 à 5, **caractérisé en ce que** ladite première partie (110) est constituée de matière plastique opaque et ladite seconde partie (130) est constituée de matière plastique transparente.

7. Dispositif (10; 100) selon l'une quelconque des revendications précédentes, **caractérisé en ce que** ladite tête de distribution (18 ; 180) est filetée de manière externe.

8. Dispositif (10; 100) selon l'une quelconque des revendications précédentes, **caractérisé en ce que** ladite ouverture traversante longitudinale (14 ; 140) possède une extrémité (16; 160), opposée à celle où la tête de distribution (18; 180) s'étend, qui est filetée pour l'accouplement amovible d'une cartouche (30 ; 300) contenant la composition de type pâte (C) à irradier.

9. Dispositif (10; 100) selon l'une quelconque des revendications précédentes, **caractérisé en ce que** ledit au moins un élément d'irradiation (24 ; 240) est une diode électroluminescente.

10. Dispositif (10; 100) selon la revendication 9, **caractérisé en ce que** ladite diode électroluminescente (24 ; 240) est une diode UV ou RVB.

11. Dispositif (10; 100) selon l'une quelconque des revendications précédentes, **caractérisé en ce que** ladite source d'énergie consiste en une ou plusieurs piles jetables ou rechargeables.

12. Cartouche (50) pour une application sur un site de traitement d'une composition de type pâte (C) à irradier, **caractérisée en ce qu'**elle comprend un tube (30 ; 300) contenant la composition de type pâte (CF) à irradier et un dispositif d'irradiation (10) selon l'une quelconque des revendications 1 à 11, ledit tube de composition de type pâte étant apte à être associé de manière amovible audit dispositif d'irradiation.

13. Cartouche (50) selon la revendication 12, **caractérisée en ce que** ladite composition de type pâte (C) est une composition de type pâte photosensible.
